# EUROPEAN PATENT SPECIFICATION

(11) **EP 2 249 782 B1**
(45) Date of publication and mention of the grant of the patent: **30.08.2017**
(21) Application number: 09715897.6
(22) Date of filing: 25.02.2009
(51) Int. Cl.: A61K 8/42, A61K 8/49, A61K 8/34, A61K 8/67, A61Q 7/00, A61Q 5/12

(54) **HAIR CARE COMPOSITIONS AND METHODS FOR INCREASING HAIR DIAMETER**
HAARPFLEGEZUSAMMENSETZUNGEN UND VERFAHREN ZUR VERGRÖSSERUNG DES HAARDURCHMESSERS
COMPOSITIONS DE TRAITEMENT CAPILLAIRE ET PROCÉDÉS D'AUGMENTATION DU DIAMÈTRE DES CHEVEUX

(30) Priority: 29.02.2008 US 67685
(43) Date of publication of application: 17.11.2010
(73) Proprietor: The Procter & Gamble Company, Cincinnati, OH 45202 (US)
(72) Inventor: IWATA, Toshiyuki, Kobe Hyogo 651-2277 (JP); TAKATA, Koji, Kobe Hyogo 658-0051 (JP); LEE, Yungi, Kobe Hyogo 658-0084 (JP); DAWSON, Thomas, Larry, Hamilton Ohio 45011 (US); FISHER, Brian, Keith, Cincinnati Ohio 45249 (US); YOUNGQUIST, Robert, Scott, Mason Ohio 45040 (US); FUENTES, Gary, Richard, Batesville Indiana 47006 (US); SUNKEL, Jorge, Max, Cincinnati Ohio 45241 (US); KOLODZIK, Martha, Jane, Aurora Indiana 47001 (US)
(74) Representative: Sauvaître, Thibault Bruno
(86) International application number: PCT/IB2009/050759
(87) International publication number: WO 2009/107072

(56) References cited:
- EP-A1- 0 325 969
- WO-A2-2008/027541
- DE-A1-102005 003 949
- FR-A1- 2 751 541
- JP-A- 2001 288 043
- US-A- 5 270 035
- US-A- 5 523 078
- ANONYMOUS: "Plantur 39 Caffeine Tonic 200ml" INERNET ARTICLE, [Online] 26 July 2008 (2008-07-26), XP002534819 Retrieved from the Internet: URL:http://www.askshop.co.uk/shopping/plan tur-39-caffeine-tonic-200ml.html> [retrieved on 2009-06-29]

## Description

### FIELD OF THE INVENTION

The present invention relates to hair care compositions and methods for increasing hair diameter.

### BACKGROUND OF THE INVENTION

Many attributes contribute to the appearance of hair considered to be attractive. For instance, hair with a full and/or thick appearance is very desirable, whether it be on the scalp, beard, or moustache regions. In contrast, hair with a thin appearance is not as attractive, and can even lead to a perception that the thin-haired individual is older than their chronological age. Furthermore, thin hair can be more difficult to style, and typically cannot be styled into as many hairstyles, leaving the individual frustrated and with an unkempt appearance. Because of the foregoing problems associated with thin hair, many thin-haired individuals expend great effort and time on grooming, yet still do not attain their desired hairstyle and appearance. This can lead to frustration and/or lack of confidence in his or her appearance. These problems can be experienced by both female and male consumers.

Accordingly, there is a need to provide consumers with a way to increase the fullness and/or thickness of hair appearance, thus resulting in younger-looking, more attractive hair appearance.

There exists a variety of products which attempt to meet the above need by, for example, increasing hair diameter. However, there still exists a need for such products providing improved hair diameter increasing benefit by improved penetration of hair diameter increasing actives. There also exists a need for such products providing improved feel during usage and/or after usage. However, it is still difficult to obtain such improved feel during usage and/or after usage, while not-compromising the penetration of the hair diameter increasing agent.

Based on the foregoing, there remains a need for hair care compositions which provide improved hair diameter increase benefit. There also remains a need for such hair care compositions which provide improved feel during usage and/or after usage, while not-compromising the penetration of the hair diameter increasing agent.

None of the existing art provides all of the advantages and benefits of the present invention.

### SUMMARY OF THE INVENTION

The present invention is directed to a hair care composition comprising:
A hair care composition comprising:
   (i) a hair diameter increasing agent comprising a xanthine compound, a vitamin B3 compound, and a panthenol compound; and
   (ii) an carrier comprising water and monohydric alcohols having 1 to 6 carbons;
   wherein the monohydric alcohol is present in the composition at a level by weight of from 30% to 90%;
   wherein said xanthine compound includes caffeine xanthine, 1-methyl xanthine, theophylline, theobromine, and mixtures thereof;
   wherein said vitamin B3 compound includes niacinamide and salts thereof, nicotinic acid and salts thereof, nicotinyl alcohol and salts thereof, nicotinic acid esters, nicotinyl amino acids, nicotinyl alcohol esters of carboxylic acids, nicotinic acid N-oxide, niacinamide N-oxide, or mixtures thereof;
   wherein said panthenol compound includes D-panthenol, DL-panthenol, panthenyl triacetate, panthetine, pantotheine, panthenyl ethyl ether, pantoyl lactose, ethyl panthenol, or mixtures thereof;
   wherein the composition further comprises a thickener being a non-cellulose based amphiphilic polymer;
   wherein the composition further comprises a substantially soluble silicone conditioning agent being a substantially soluble silicone copolyol conditioning agent;
   wherein the composition includes 0.1% or less by weight of substantially insoluble oily compounds.

The present invention is also directed to a method as recited in claim 11 for increasing hair diameter, comprising topically applying the above composition to a region where hair diameter increase is desired.

The hair care compositions and methods of the present invention provide improved hair diameter increase benefit. The hair care compositions and methods of the present invention also provide improved feel during usage and/or after usage, while not-compromising the penetration of the hair diameter increasing agent.

These and other features, aspects, and advantages of the present invention will become better understood from a reading of the following description, and appended claims.

### DETAILED DESCRIPTION OF THE INVENTION

While the specification concludes with the claims particularly pointing and distinctly claiming the invention, it is believed that the present invention will be better understood from the following description.

All percentages, parts and ratios are based upon the total weight of the hair care compositions of the present invention and all measurements made are at 25°C, unless otherwise specified. All such weights as they pertain to listed ingredients are based on the active level and, therefore, do not include carriers or by-products that may be included in commercially available materials, unless otherwise specified.

Herein, "comprising" means that other steps and other ingredients which do not affect the end result can be added. This term encompasses the terms "consisting of" and "consisting essentially of".

Herein, "mixtures" is meant to include a simple combination of materials and any compounds that may result from their combination.

Herein, "hair diameter increase" and "increasing hair diameter" mean increasing diameters of new hairs. New hairs herein means, any portions of hairs which are newly generated in hair follicles and appeared over the surface of skin/scalp as a result of hair growth which include, for example, tips of newly growing hairs and roots of existing hairs.

All measurements are understood to be made at 25°C and at ambient conditions, where "ambient conditions" means conditions under one atmosphere of pressure and at 50% relative humidity.

### HAIR CARE COMPOSITIONS

Hair care compositions of the present invention comprise:
(i) a hair diameter increasing agent comprising a xanthine compound, a vitamin B3 compound, and a panthenol compound; and
(ii) an carrier comprising water and monohydric alcohols having 1 to 6 carbons;
wherein the monohydric alcohol is present in the composition at a level by weight of from 10% to 90%., preferably from 30% to 90%; and
wherein the composition further comprises: a substantially soluble silicone conditioning agent, being a substantially soluble silicone copolyol conditioning agent; and a thickener, preferably Acrylates/C10-30 Alkyl Acrylate Crosspolymer.

It is believed that the use of monohydric alcohol at a level of from 10% to 90%, especially at a level of from 30% to 90%, provides clean usage feel and improved solubilization and penetration of hair diameter increasing agents especially caffeine, niacinamide and pantehnol.

It is also believed that the use of substantially soluble silicone conditioning agent, especially a substantially soluble silicone copolyol conditioning agent, provide conditioning benefits, clean dry feel, while not-compromising the penetration of hair diameter increasing agents.

It is further believed that the use of thickener provide better usage feel such that the composition does not drop undesirably. Among the thickeners, Acrylates/C10-30 Alkyl Acrylate Crosspolymer is preferred especially when the composition comprises higher level of alcohols such as ethanol in view of better solubility in such compositions.

As used herein, the term "hair care compositions" are compositions that are applied to the hair and/or the skin underneath the hair, including compositions used to treat or care for the hair. Products contemplated by the phrase "hair care composition" include, but are not limited to liquids, creams, wipes, hair conditioners (rinse-off and leave-on), hair and/or scalp tonics, shampoos, hair colorants, mousses, propellant lotions, emulsions, shave gels, after-shave tonics and lotions, temporary beard hair dyes, and the like. Among the above hair care compositions, leave-on hair care compositions are preferred, in view of providing effectively delivering the benefits of the present invention especially hair diameter increase benefit. Such leave-on hair care compositions include, for example, leave-on hair conditioning compositions, hair and/or scalp tonics, and hair styling compositions. Such leave-on hair care compositions can have a pH of, for example, from 3 to 7.5, preferably from 3.5 to 7, more preferably from 4 to 7.

In the present invention, it may be preferred that the composition is in a non-emulsion form. It is believed that, the hair diameter increasing agent of the present invention provide improved performance to scalp, skin and/or hair follicles, when it is contained in a non-emulsion composition rather than in an emulsion composition.

Preferably, the compositions of the present invention are substantially free of substantially insoluble oily compounds. It is believed that lower levels of such substantially insoluble oily components provide at least one of the following:
improved penetration of hair diameter increasing agents to scalp, skin, and/or hair follicles;
improved solid particulate formation of the deposition agent, especially when the deposition agents are those being soluble in the composition and forming solid particulate on the hair; and
improved usage feel such as improved clean feel, especially when the composition is for leave-on use.

In the present invention, the compositions being "substantially free" of substantially insoluble oily compound means that the composition includes 1.0% or less, preferably 0.5% or less, more preferably 0.1% or less, still more preferably 0.05% or less, even more preferably 0% by weight of substantially insoluble oily compounds. By "substantially insoluble" ingredients, what is meant in the present invention is that: the ingredient is substantially insoluble in the compositions at the level used; and, when containing the ingredient at the level used, the composition has a transmittance of below 25%, preferably below 35%, more preferably below 50%, still more preferably below 60%, even more preferably below 70%, and further more preferably below 80% at 25°C. The transmittances are measured at 600nm using UV-1601 which is a UV-visible spectrophotometer available from Shimadzu. Such "substantially insoluble" oily compounds are typically those selected from hydrocarbons, fatty compounds, and mixtures thereof. Such hydrocarbons include, for example, poly α-olefin oils, paraffins, waxes, and mixtures thereof. Such fatty compounds include, for example, fatty alcohols such as cetyl alcohol and stearyl alcohol, fatty acids such as stearic acid, fatty alcohol derivatives and fatty acid derivatives such as esters and ethers thereof, and mixtures thereof.

### A. Hair diameter increasing agent

The composition of the present invention comprises a hair diameter increasing agent. A safe and effective amount of the hair diameter increasing agent can be included in the composition. The term "safe and effective amount," as used herein, means an amount of a compound or composition sufficient to increase the diameter of the hair in the subject region of hair, when used for a result-effective period of time, but low enough to avoid serious side effects, i.e., to provide a reasonable benefit to risk ratio, within the scope of sound judgment of the skilled artisan. For example, the hair diameter increasing agent can be included in the composition at a level by weight of the composition of, from 0.1%, preferably from 0.3%, more preferably from 0.5%, and to 20%, preferably to 15%, more preferably to 10%, in view of usage feel and hair diameter increasing effect.

Hair diameter increasing agents useful herein are those being substantially soluble in the composition, in order to provide hair diameter increasing benefit by penetrating into scalp, skin and/or hair follicles. By "substantially soluble" ingredients, what is meant in the present invention is that: the ingredient is substantially soluble in the compositions at the level used; and, when containing the ingredient at the level used, the composition has a transmittance of 25% or more, preferably 35% or more, more preferably 50% or more, still more preferably 60% or more, even more preferably 70% or more, and further more preferably 80% or more, at 25°C.

The hair diameter increasing agent of the present invention comprises a xanthine compound, a vitamin B3 compound, and a panthenol compound. Preferably, the hair diameter increasing agent comprises caffeine, niacinamide, and panthenol. The use of the three compounds as a mixture, compared to the use of each compound, provide improved hair diameter increasing benefit.

### A-1. Xanthine compound

As used herein, "xanthine compound" means one or more xanthines, derivatives thereof, and mixtures thereof. Xanthine Compounds of the hair care composition include caffeine, xanthine, 1-methyl xanthine, theophylline, theobromine, and mixtures thereof. Among these compounds, caffeine is preferred in view of its solubility in the composition and hair diameter increasing effect. The composition can contain from 0.1%, preferably from 0.2%, more preferably from 0.3%, still more preferably from 0.4%, and to 10%, preferably to 5%, more preferably to 2% by weight of a xanthine compound by weight of the composition, in view of usage feel and hair diameter increasing effect.

### A-2. Vitamin B3 compound

As used herein, "vitamin B3 compound" means a one or more compounds having the formula: 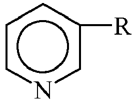 wherein R is - CONH₂ (i.e., niacinamide), - COOH (i.e., nicotinic acid) or - CH₂OH (i.e., nicotinyl alcohol); derivatives thereof; mixtures thereof; and salts of any of the foregoing.

Derivatives of the foregoing vitamin B3 compounds include nicotinic acid esters, including non-vasodilating esters of nicotinic acid (e.g, tocopherol nicotinate, and myristyl nicotinate), nicotinyl amino acids, nicotinyl alcohol esters of carboxylic acids, nicotinic acid N-oxide and niacinamide N-oxide.

One or more vitamin B3 compounds may be used herein. Preferred vitamin B3 compounds are niacinamide and tocopherol nicotinate. Niacinamide is more preferred, in view of usage feel, its solubility in the composition, and hair diameter increasing effect. It is believed that, niacinamide can stay on scalp, pore and hair, where cells contribute to hair diameter increase exist, for a longer period of time and thus can provide pro-longed benefit, while other vitamine B3 products such as nicotinates may penetrate fast into scalp, and thus may move away from such cells. It is also believed that, niacinamide can be stable in the composition and when applied for a longer period of time, while other vitamine B3 products such as nicotinic acid esters may be decomposed to nicotinic acids, and thus may cause skin flushing and itchiness. Such nicotinic acid esters include, for example, ethyl nicotinate, octyl nicotinate, myristyl nicotinate, and cetyl nicotinate. When used, salts, derivatives, and salt derivatives of niacinamide are preferably those having substantially the same efficacy as niacinamide.

In a preferred embodiment, the ring nitrogen of the vitamin B3 compound is substantially chemically free (e.g., unbound and/or unhindered), or after delivery to the skin becomes substantially chemically free ("chemically free" is hereinafter alternatively referred to as "uncomplexed"). More preferably, the vitamin B3 compound is essentially uncomplexed. Therefore, if the composition contains the vitamin B3 compound in a salt or otherwise complexed form, such complex is preferably substantially reversible, more preferably essentially reversible, upon delivery of the composition to the skin. For example, such complex should be substantially reversible at a pH of from 5.0 to 6.0. Such reversibility can be readily determined by one having ordinary skill in the art.

More preferably the vitamin B3 compound is substantially uncomplexed in the composition prior to delivery to the keratinous tissue. Exemplary approaches to minimizing or preventing the formation of undesirable complexes include omission of materials which form substantially irreversible or other complexes with the vitamin B3 compound, pH adjustment, ionic strength adjustment, the use of surfactants, and formulating wherein the vitamin B3 compound and materials which complex therewith are in different phases. Such approaches are well within the level of ordinary skill in the art.

Thus, in a preferred embodiment, the vitamin B3 compound contains a limited amount of the salt form and is more preferably substantially free of salts of a vitamin B3 compound. Preferably the vitamin B3 compound contains less than 50% of such salt, and is more preferably essentially free of the salt form. The vitamin B3 compound in the compositions hereof having a pH of from 4 to 7 typically contain less than 50% of the salt form.

The composition can contain from 0.1%, preferably from 0.3%, more preferably from 0.5%, still more preferably from 1%, and to 25%, preferably to 15%, more preferably to 7.5% by weight of a vitamin B3 compound by weight of the composition, in view of usage feel and hair diameter increasing effect.

### A-3. Panthenol compound

As used herein, the term "panthenol compound" is broad enough to include panthenol, one or more pantothenic acid derivatives, and mixtures thereof. Panthenol and its derivatives can include D-panthenol ([R]-2,4-dihydroxy-N-[3-hydroxypropyl)]-3,3-dimethylbutamide), DL-panthenol, pantothenic acids and their salts, preferably the calcium salt, panthenyl triacetate, panthetine, pantotheine, panthenyl ethyl ether, pantoyl lactose, or mixtures thereof.

Compositions comprising pantothenic acid derivatives that remain more stable than panthenol and other similar materials in acidic compositions or in compositions containing acid-producing materials such as aluminum-containing actives, can also be suitable for use herein. The selected pantothenic acid derivatives are most typically in liquid form and dispersed throughout or otherwise solubilized within the liquid carrier component of the composition.

The term "pantothenic acid derivative" as used herein refers to those materials that conform to the formula: 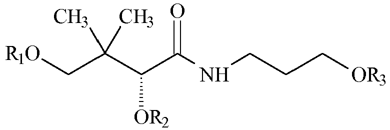 wherein R₁, R₂ and R₃ are hydrogen, C2-C20 hydrocarbons, C2-C20 carboxylic acid esters, or combinations thereof, provided that not more than two of R₁, R₂ and R₃ are hydrogen. In one embodiment, R₁, R₂ and R₃ are independently selected from hydrogen, C2-C8 hydrocarbons, C2-C8 carboxylic acid esters, or combinations thereof; in another embodiment, R₁ and R₂ are hydrogen, and R₃ is a C2-C8 hydrocarbon, C2-C8 carboxylic acid ester, or combinations thereof; in yet another embodiment, R₁ and R₂ are hydrogen and R₃ is ethyl. The selected pantothenic acid derivatives may be derived or otherwise obtained from any known source, which may include pantothenic acid or materials other than pantothenic acid, so long as the resulting material has the above defined chemical formula.

Specific non-limiting examples of selected pantothenic acid derivatives for use herein include ethyl panthenol, panthenyl triacetate, and combinations thereof. In a particular embodiment, a pantothenic acid derivative comprises the d-isomeric form(s) of such derivative form(s), such as d-ethyl panthenol.

Among these compounds, D-panthenol is preferred in view of its solubility in the composition and hair diameter increasing effect. The composition can contain from 0.01%, preferably from 0.02%, more preferably from 0.05%, and to 3%, preferably to 1%, more preferably to 0.5% by weight of a panthenol compound, by weight of the composition in view of usage feel and hair diameter increasing effect.

### B. Carrier

The composition further comprises a carrier comprising water and monohydric alcohols. The lower alkyl alcohols useful herein are monohydric alcohols having 1 to 6 carbons, preferably ethanol and isopropanol, more preferably ethanol. Generally, the compositions of the present invention comprise from 10%, preferably from 30%, more preferably from 80%, and to 99%, preferably to 95%, more preferably to 95% by weight of such carriers. Such alcohols such as ethanol is included at a level by weight of the composition, of from 10%, preferably from 20%, more preferably from 30%, still more preferably from 40%, and to 90%, preferably to 70%, more preferably to 60%, in view of providing clean usage feel, improved solubilization and penetration of hair diameter increasing agents especially caffeine, niacinamide and panthenol.

### C. Thickener

Preferably, the compositions further comprise a thickener for better usage feel such that the composition does not drip undesirably onto other areas of the body, onto clothing, or onto home furnishings. Such compositions can have a zero shear viscosity of from 0.1 Pa·s, preferably from 10 Pa·s, more preferably from 100Pa·s, and to 100,000 Pa·s, preferably to 50,000Pa·s, more preferably to 10,000Pa·s. Generally, the viscosity of the personal care composition decreases according to the increase of shear stress. At a lower shear stress, the viscosity of the composition does not change and this is called "First Newtonian Plateau". In the shear stress range higher than a yield point, the viscosity of the composition decreases. The zero shear viscosity is the average of the viscosity measured in the first Newtonian plateau. The zero shear viscosity is measured at 25°C by shear stress ramp mode using AR2000 available from TA Instruments.

Preferred thickeners herein are, for example, non-cellulose based amphiphilic polymers which comprise at least one fatty chain and non-cellulose based hydrophilic moieties, and which are preferably crosslinked. Such non-cellulose based amphiphilic polymers include, for example, carboxylic acid/carboxylate copolymers which are obtained by copolymerizing: 1) a carboxylic acid monomer such as acrylic acid, methacrylic acid, maleic acid, maleic anhydride, itaconic acid, fumaric acid, crotonic acid, or α-chloroacrylic acid, preferably acrylic acid; 2) a carboxylic ester having an alkyl chain of from 1 to 30 carbons, preferably acrylic esters having an alkyl chain of from 10 to 30 carbons; and preferably 3) a crosslinking agent such as allyl ethers of sucrose and allyl ethers of pentaerythritol. Highly preferred examples of such non-cellulose based amphiphilic polymers include Acrylates/C10-30 Alkyl Acrylate Crosspolymer.

Preferred are those being substantially soluble in the composition, in view of not-compromising the penetration of hair diameter increasing agent. In the present invention, it may be preferred that the composition is substantially free of substantially insoluble thickeners. When the composition comprises higher level of alcohols such as ethanol, such substantially insoluble thickeners may be cellulose based thickeners such as cellulose and modified celluloses, and gum thickeners such as xanthan gum and guar gum. In the present invention, the compositions being "substantially free" of substantially insoluble thickeners means that the composition includes 0.5% or less, preferably 0.1% or less, more preferably 0.05% or less, still more preferably 0.03% or less, even more preferably 0% by weight of substantially insoluble thickeners.

In the present invention, Acrylates/C10-30 Alkyl Acrylate Crosspolymer is preferred especially when the composition comprises higher level of alcohols such as ethanol, in view of better solubility in such compositions compared to other anionic/nonionic thickeners such as carbomer and cellulose polymers. Acrylates/C10-30 Alkyl Acrylate Crosspolymer is also preferred in view of hair feel when dried, compared to cationic/nonionic thickening polymers. Acrylates/C10-30 Alkyl Acrylate Crosspolymer can be used together with any neutralizer such as triethanolamine.

Such thickeners can be included in the composition, at a level by weight of the composition of from 0.01%, preferably from 0.05%, and to 5%, preferably to 3%, more preferably to 1%, in view of thickening effect, feel during and/or after usage, and/or solubility in the composition.

### D. Substantially soluble silicone conditioning agent

Preferably, the composition further comprises a substantially soluble silicone conditioning agent. Such silicone conditioning agents can be included in the composition, at a level by weight of the composition of from 0.05%, preferably from 0.1%, more preferably from 0.2%, and to 10%, preferably to 5%, more preferably to 2%, in view of conditioning benefit and clean dry feel.

Such silicone conditioning agents useful herein are composition, for example: silicone copolyol conditioning agents; and anionic silicone conditioning agents such as AcrylatesBehenyl Acrylate/Dimethicone Methacrylate Copolymer, Actylic Acid/Stearyl Methacrylate/Dimethicone Methacrylate Copolymer. Preferably, silicone conditioning agents useful herein are silicone copolyol conditioning agents. It is believed that, compared to other soluble conditioning compounds such as fatty esters and fatty ethers, substantially silicone conditioning agents especially substantially soluble silicone copolyol conditioning agents can provide conditioning benefits while providing better clean dry feel and not-compromising the penetration of hair diameter increasing agent.

Among such silicone copolyol conditioning agents, preferred are nonionic silicone copolyol conditioning agents, especially when thickener is an anionic polymer such as Acrylates/C10-30 Alkyl Acrylate Crosspolymer, in view of compatibility with such thickeners. Such nonionic silicone copolyol conditioning agents are those being substantially free of cationic, amido and amino substitutions. What meant by being "substantially free of cationic, amido and amino substitutions" is that the silicone compounds contains 1% or less, preferably 0.5% or less, more preferably 0% by weight of such substitutions.

Such silicone copolyols useful herein include, for example, dimethicone copolyols dimethicone backbone and polyoxyalkylene substitutions such as polyethylene oxide or/and polypropylene oxide substitutions which are grafted to the middle and/or the ends of the dimethicone backbone. In view of the solubility in the compositions, graft silicone copolyols are preferred compared to silicone copolyols those having the substitutions only at the ends of the backbone. The grafted silicone copolyols useful herein have an HLB value of preferably from 5 to 17, more preferably from 8 to 17, still more preferably from 8 to 12. The grafted silicone copolyols having the same INCI name have a variety of the weight ratio, depending on the molecular weight of the silicone portion and the number of the polyethylene oxide or/and polypropylene oxide substitutions.

Commercially available grafted dimethicone copolyols include, for example: those having a tradename Silsoft 430 having an HLB value of from 9 to 12 (INCI name "PEG/PPG-20/23 dimethicone") available from Momentive Performance Materials; those having a tradename Silsoft 475 having an HLB value of from 13 to 17 (INCI name "PEG-23/PPG-6 dimethicone"); those having a tradename Silsoft 880 having an HLB value of from 13 to 17 (INCI name "PEG-12 dimethicone"); those having a tradename Silsoft 440 having an HLB value of from 9 to 12 (INCI name "PEG-20/PPG-23 dimethicone"); those having a tradename DC5330 (INCI name "PEG-15/PPG-15 dimethicone") available from Dow Corning. Among them, preferred are those having a tradename Silsoft 430 having an HLB value of from 9 to 12 (INCI name "PEG/PPG-20/23 dimethicone") available from Momentive Performance Materials.

### E. Optional Ingredients

The composition of the present invention may include other optional ingredients, which may be selected by the artisan according to the desired characteristics of the final product and which are suitable for rendering the composition more cosmetically or aesthetically acceptable or to provide them with additional usage benefits. Such optional ingredients can be incorporated into the composition at a level of from 0.01% to 10%, preferably from 0.05% to 5% by weight of the composition.

The hair care compositions of the present invention may further contain an additional benefit agent. Such additional benefit agent is selected from the group consisting of: hair volume-up agents such as film forming polymers; hair conditioning agents such as cationic surfactants, cationic polymers, silicones and fatty compounds; scalp sensation agents; hair color agents; scent agents; and mixtures thereof.

A wide variety of other additional components can be formulated into the present compositions. These include: hair growth agents such as minoxidil; anti-dandruff agents such as zinc pyrithione; conditioning agents such as hydrolysed collagen, vitamins, hydrolysed keratins, proteins, plant extracts, and nutrients; preservatives such as benzyl alcohol, methyl paraben, propyl paraben and imidazolidinyl urea; pH adjusting agents, such as citric acid, sodium citrate, succinic acid, phosphoric acid, sodium hydroxide, sodium carbonate; salts, in general, such as potassium acetate and sodium chloride; coloring agents, such as any of the FD&C or D&C dyes; perfumes; and sequestering agents, such as disodium ethylenediamine tetra-acetate; and ultraviolet and infrared screening and absorbing agents such as octyl salicylate.

### METHOD OF USE / METHOD FOR INCREASING HAIR DIAMETER

The present invention also provides a method for increasing the diameter of the hair. In one aspect, the method comprises topically applying the composition of the present invention, to a region where hair diameter increase is desired. For instance, the hair care composition can be applied to the scalp and/or face (e.g., beard or moustache area). The region of hair can be located on any part of the body. For instance, it can grow from a skin surface located on at least a portion of the scalp or the face.

It is preferred that an effective amount of a composition of the present invention is topically applied to the desired region at effective frequency over a result-effective period of time.

The term "effective amount," as used herein, means an amount of a compound or composition sufficient to increase the diameter of the hair in the subject region of hair. It depends on the product form and concentration of the hair diameter increasing agent. For example, the composition can be applied at an amount of from 0.1ml to 10ml to the subject regions.

The term "effective frequency" as used herein means a frequency to apply the composition sufficient to increase the diameter of the hair in the subject region of hair. It depends on the product form and concentration of the hair diameter increasing agent. For example, the composition can be applied at least once a few week, more preferably at least once a week, more preferably at least once a day, and up to three times a day.

The term "result-effective period of time," as used herein, means a period of time sufficient to increase the diameter of the hair in the subject region of hair. It depends on the product form and concentration of the hair diameter increasing agent. For example, the composition can be applied for at least a week, more preferably at least two weeks, still more preferably at least four weeks.

In still another embodiment, the method comprises applying the composition according to a regimen, wherein said regimen comprises:
(a) cleansing the scalp and/or face to form a cleansed scalp and/or face;
(b) topically applying the composition to said cleansed scalp and/or cleansed face.

### EXAMPLES

The following examples further describe and demonstrate embodiments within the scope of the present invention. The examples are given solely for the purpose of illustration and are not to be construed as limitations of the present invention, as many variations thereof are possible without departing from the spirit and scope of the invention. Where applicable, ingredients are identified by chemical or CTFA name, or otherwise defined below.

### Example 1: Clinical Study

A composition comprising a mixture of Caffeine, Niacinamide and Panthenol demonstrated a clinically significant increase in hair width versus a placebo. In this study, a scalp tonic containing 5% Niacinamide, 1.5% Caffeine, and 0.3% D-Panthenol in a water/alcohol matrix (25% ethanol, q.s. water) was evaluated. This formula also included 0.3% hydroxypropylmethylcellulose as a thickening agent. In this split-head study, 1.5 ml of the tonic was applied to one side of the head two times per day, and 1.5ml of the placebo was applied to the other side of the head two times daily.

This was a 14 week, double blinded, randomized, split-head and controlled clinical study in women ages 18-65 (inclusive) who perceived themselves as having thinning hair. This study consisted of a 2 week pre-treatment phase (use of placebo on both sides of the head to learn proper product application methods prior to the actual treatment phase) followed by a 12 week treatment phase.

Upon arriving at the test site, subjects were acclimated in an environmentally controlled room (70°F ± 2 and 30-45% relative humidity) for 30 minutes prior to having their scalp evaluated by a qualified skin grader to assess skin health. After subjects had their scalp assessed by a qualified skin grader, a licensed cosmetologist identified two scalp sites for clipping (areas of approximately 1.8 cm x 1.8 cm), one on the top left lateral scalp, approximately 4-5" back into the hairline and the other was symmetrically placed on the top right lateral scalp. A template was used to mark the 4 corners and a notch slightly below center of each treatment site. The clipped sites were approximately 2" lateral to either of their respective sides from the midline of the head/scalp. The licensed cosmetologist then cut the hair within each of the scalp clipping sites with a small battery operated hair clipper to 1mm in length. After the sites were prepared, a trained clinical assistant placed a small, permanent dot tattoo approximately 1 mm above the marked notch (located slightly below center of the treatment site) on each treatment site to provide a reference for identification of these sites for digital imaging that was performed at designated study time points.

For Baseline, Week 4, 8, and 12, the clipped scalp sites were identified using the template and marking the 4 corners of the template with a Sharpie® marker or equivalent. The licensed cosmetologist then evenly clipped the short hairs within the 2 clipped sites to 1 mm as previously conducted. Following hair clipping, a technician captured a baseline image of each clipped scalp site using a Hi-Scope® digitized imaging system. The tattoo that was placed within each lower treatment site was used as a reference point to position the image for capture and storage. This baseline image was used when blending post-treatment images during the study. The next day, approximately 24 hours following the hair clipping visit, a technician captured an image of each lower scalp clipping site using a Hi-Scope® digitized imaging system. The tattoo that was placed within each lower treatment site was used as a reference point to position the image for capture and storage after aligning the hair follicle openings with the image captured at Baseline, Day 1. This procedure was used for Week 4, 8 and 12.

A significant increase in hair diameter was observed for the hair tonic treated hair vs. the placebo-treated hair in the human clinical test. The Change from Baseline Adjusted Mean for the tonic-treated hair (0.663µm) is larger than that of the placebo-treated (0.165µm). As this is a split-head design, subject variation was greatly minimized. Additionally, since the exact same hairs were imaged throughout the study, variations in width due to measuring different hairs through the study were greatly minimized.

**Example 1: Clinical Study - Comparison of treatment effect**

| N | Treatment | Grouping | Change from Baseline Adjusted Mean (µm) |
|---|---|---|---|
| 40 | Placebo (Tonic Vehicle) | A | 0.165 |
| 40 | Tonic Product | B | 0.663 |

An image analysis algorithm was used to measure the hair diameter from the digital micrographs. The algorithm automatically carried out several steps to isolate and characterize hairs in each digital image. First, the algorithm performed a local background leveling function and enhancement of the red component in the color image. Automatic thresholding of the color image was performed to obtain a binary image (black and white) of the hairs on the background. The algorithm then identified hairs as continuous black lines and broke the lines into individual hairs at branch points and points of high curvature. Next, the algorithm eliminated very short objects and retained the remaining objects as 'hairs'. In the final step, the algorithm measured and recorded position and diameter (width) of each detected hair.

The hair system image acquisition and analysis methods used herein were the methods published by Berger, R.S. et al., in 2003 in "The effects of minoxidil, 1% pyrithione zinc and a combination of both on hair density: a randomized controlled trial. British Journal of Dermatology, 149, 354-362," with the exception that for the study of this example, different treatments, scalp imaging sites, and timepoints were used.

### Example 2: Synergy study

A mixture of Caffeine, Niacinamide and Panthenol demonstrated a surprising increase in the survival of metabolically stressed dermal papilla cells, versus individual components and versus mixtures of two of them, as shown below. The dermal papilla, in normal human hair, is the control center for hair diameter. Increasing survival of dermal papilla cells in situ leads to increased hair diameter. Accordingly, an increase in survival of dermal papilla cells correlates with an increase in hair diameter.

Primary human dermal papilla cells were metabolically stressed for 48 hours in reduced standard tissue culture media. During this period of stress the cells were treated with the mixtures or individual ingredients below. After 48 hr the metabolic activity of the cells was measured by the amount of ATP using the Cell Titer Glo™ kit (Promega).

| Treatment | Survival rate: % Change from Control |
|---|---|
| Control (100% water) | - |
| Niacinamide (0.006%) | - 8% |
| Panthenol (0.15%) | + 6% |
| Caffeine (0.05%) | + 41% |
| Mixture of Niacinamide (0.006%) and Panthenol (0.15%) | + 22% |
| Mixture of Niacinamide (0.006%) and Caffeine (0.05%) | + 82% |
| Mixture of Panthenol (0.15%) and Caffeine (0.05%) | + 93% |
| Mixture of Niacinamide (0.006%), Panthenol (0.15%), and Caffeine (0.05%) | + 155% |

### Example 3: Hair care composition examples

### Tonics

| Component (Active weight %) | A | B | C |
|---|---|---|---|
| Caffeine | 1.5 | 0.6 | - |
| Caffeine Carboxylic Acid | - | - | 1.00 |
| Niacinamide | 5.0 | 2.0 | - |
| Tocopherol Nicotinate | - | - | 1.0 |
| Panthenol | 0.3 | 0.1 | - |
| Panthenyl Ethyl Ether | - | - | 0.2 |
| PEG/PPG-20/23 Dimethicone | - | 0.7 | 0.5 |
| Carbomer | 0.1 | - | - |
| Acrylates/C10-30 Alkyl Acrylate Crosspolymer | - | 0.3 | 0.3 |
| Triethanolamine | 0.2 | 0.04 | 0.04 |
| Ethanol | 25.0 | 50.0 | 50.0 |
| Perfume | 0.1 | 0.1 | 0.5 |
| Tocopherol | - | - | 0.1 |
| Ascorbic Acid | - | - | 0.05 |
| Pentapeptide-2 | - | - | 0.05 |
| Deionized water | Qs | Qs | Qs |

### Method of Preparation

The compositions of Examples A through C as shown above can be prepared by any conventional method well known in the art. They are suitably made as follows:
The polymeric materials, if present, are dispersed in water at room temperature, mixed by vigorous agitation. Ethanol, if included, is added to the mixture. The hair diameter increasing agents, deposition agents, adhesive agents are added to the mixture with agitation to dissolve or disperse such components. Then the remaining components such as silicones and perfumes, if included, are added to the mixture with agitation.

Examples A through C are hair care compositions of the present invention which are particularly useful for leave-on use. The compositions are topically applied to a region where hair diameter increase is desired. These examples have many advantages. For example, they provide improved hair diameter increase benefit. They also provide improved feel during usage and/or after usage, while not-compromising the penetration of the hair diameter increasing agent.

## Claims

1. A hair care composition comprising:
(i) a hair diameter increasing agent comprising a xanthine compound, a vitamin B3 compound, and a panthenol compound; and
(ii) an carrier comprising water and monohydric alcohols having 1 to 6 carbons;
wherein the monohydric alcohol is present in the composition at a level by weight of from 30% to 90%;
wherein said xanthine compound is selected from caffeine xanthine, 1-methyl xanthine, theophylline, theobromine, and mixtures thereof;
wherein said vitamin B3 compound is selected from niacinamide and salts thereof, nicotinic acid and salts thereof, nicotinyl alcohol and salts thereof, nicotinic acid esters, nicotinyl amino acids, nicotinyl alcohol esters of carboxylic acids, nicotinic acid N-oxide, niacinamide N-oxide, and mixtures thereof;
wherein said panthenol compound is selected from D-panthenol, DL-panthenol, panthenyl triacetate, panthetine, pantotheine, panthenyl ethyl ether, pantoyl lactose, ethyl panthenol, and mixtures thereof;
wherein the composition further comprises a thickener being a non-cellulose based amphiphilic polymer;
wherein the composition further comprises a substantially soluble silicone conditioning agent being a substantially soluble silicone copolyol conditioning agent;
wherein the composition includes 0.1% or less by weight of substantially insoluble oily compounds.

2. The composition of claim 1, wherein the monohydric alcohol is selected from the group consisting of ethanol, isopropanol, and mixtures thereof.

3. The composition of any of Claims 1-2, wherein the monohydric alcohol is ethanol.

4. The composition of any of Claims 1-3, wherein the xanthine compound is present in the composition at a level of from 0.1% to 10% by weight of the composition, the vitamin B3 compound is present in the composition at a level of from 0.1% to 25% by weight of the composition, and the panthenol compound is present in the composition at a level of from 0.01% to 3% by weight of the composition.

5. The composition of any of Claims 1-4, wherein the xanthine compound is caffeine, the vitamin B3 compound is niacinamide, and a panthenol compound is panthenol.

6. The composition of Claims 1 to 5, wherein the thickener is Acrylates/C10-30 Alkyl Acrylate Crosspolymer.

7. The composition of Claims 1-6, wherein the composition further contains an additional benefit agent selected from the group consisting of hair volume-up agents, hair conditioning agents, cationic polymers, silicone and fatty compounds, scalp sensation agents, hair color agents, scent agents, or mixtures thereof.

8. The composition of Claims 1-7, wherein the composition further comprises other additional components selected from the group consisting of hair growth agents, anti-dandruff agents, conditioning agents, vitamins, hydrolysed keratins, proteins, plant extracts, nutrients, preservatives, pH adjusting agents, salts, coloring agents, perfumes, sequestering agents, ultraviolet and infrared screening and absorbing agents, or mixtures thereof.

9. The composition of Claims 1-8, wherein the composition is in non-emulsion form.

10. The composition of any of Claims 1-9, wherein the composition is for leave-on use.

11. A method for increasing hair diameter, comprising topically applying a composition to a region where hair diameter increase is desired; wherein the composition is a hair care composition comprising:
(i) a hair diameter increasing agent comprising a xanthine compound, a vitamin B3 compound, and a panthenol compound; and
(ii) an carrier comprising water and monohydric alcohols having 1 to 6 carbons;
wherein the monohydric alcohol is present in the composition at a level by weight of from 30% to 90%;
wherein said xanthine compound is selected from caffeine xanthine, 1-methyl xanthine, theophylline, theobromine, and mixtures thereof;
wherein said vitamin B3 compound is selected from niacinamide and salts thereof, nicotinic acid and salts thereof, nicotinyl alcohol and salts thereof, nicotinic acid esters, nicotinyl amino acids, nicotinyl alcohol esters of carboxylic acids, nicotinic acid N-oxide, niacinamide N-oxide, and mixtures thereof;
wherein said panthenol compound is selected from D-panthenol, DL-panthenol, panthenyl triacetate, panthetine, pantotheine, panthenyl ethyl ether, pantoyl lactose, ethyl panthenol, and mixtures thereof;
wherein the composition further comprises a thickener being a non-cellulose based amphiphilic polymer;
wherein the composition further comprises a substantially soluble silicone conditioning agent being a substantially soluble silicone copolyol conditioning agent; and
wherein the composition includes 0.1% or less by weight of substantially insoluble oily compounds.

12. The use of a composition for increasing hair diameter; wherein the composition is a hair care composition comprising:
(i) a hair diameter increasing agent comprising a xanthine compound, a vitamin B3 compound, and a panthenol compound; and
(ii) an carrier comprising water and monohydric alcohols having 1 to 6 carbons;
wherein the monohydric alcohol is present in the composition at a level by weight of from 30% to 90%;
wherein said xanthine compound is selected from caffeine xanthine, 1-methyl xanthine, theophylline, theobromine, and mixtures thereof;
wherein said vitamin B3 compound is selected from niacinamide and salts thereof, nicotinic acid and salts thereof, nicotinyl alcohol and salts thereof, nicotinic acid esters, nicotinyl amino acids, nicotinyl alcohol esters of carboxylic acids, nicotinic acid N-oxide, niacinamide N-oxide, and mixtures thereof;
wherein said panthenol compound is selected from D-panthenol, DL-panthenol, panthenyl triacetate, panthetine, pantotheine, panthenyl ethyl ether, pantoyl lactose, ethyl panthenol, and mixtures thereof.

## Patentansprüche

1. Haarpflegezusammensetzung, umfassend:
(i) ein Mittel zur Erhöhung des Haardurchmessers, das eine Xanthin-Verbindung, eine Vitamin-B3-Verbindung und eine Panthenol-Verbindung umfasst; und
(ii) einen Träger, der Wasser und einwertige Alkohole mit 1 bis 6 Kohlenstoffen umfasst;
wobei der einwertige Alkohol in der Zusammensetzung in einer Konzentration von 30 bis 90 Gew.-% vorhanden ist.
wobei die Xanthin-Verbindung aus Koffein-Xanthin, 1-Methyl-Xanthin, Theophyllin, Theobromin und deren Mischungen ausgewählt ist;
wobei die Vitamin-B3-Verbindung aus Niacinamid und dessen Salzen, Nicotinsäure und deren Salzen, Nicotinylalkohol und dessen Salzen, Nicotinsäureestern, Nicotinylaminosäuren, Nicotinylestern von Carbonsäuren, Nicotinsäure-N-oxid, Niacinamid-N-oxid und deren Mischungen ausgewählt ist;
wobei die Panthenol-Verbindung aus D-Panthenol, DL-Panthenol, Panthenyltriacetat, Panthetin, Pantothein, Panthenylethylether und deren Mischungen ausgewählt ist;
wobei die Zusammensetzung ferner ein Verdickungsmittel umfasst, bei dem es sich um ein nicht auf Cellulose basierendes amphiphiles Polymer handelt;
wobei die Zusammensetzung ferner ein im Wesentlichen lösliches Silikon-Konditioniermittel umfasst, bei dem es sich um ein im Wesentlichen lösliches Silikoncopolyol-Konditioniermittel handelt;
wobei die Zusammensetzung 0,1 Gew.-% oder weniger im Wesentlichen unlöslicher öliger Verbindungen enthält.

2. Zusammensetzung nach Anspruch 1, wobei der einwertige Alkohol aus der Gruppe bestehend aus Ethanol, Isopropanol und deren Mischungen ausgewählt ist.

3. Zusammensetzung nach einem der Ansprüche 1 bis 2, wobei der einwertige Alkohol Ethanol ist.

4. Zusammensetzung nach einem der Ansprüche 1-3, wobei die Xanthin-Verbindung in der Zusammensetzung in einer Konzentration von 0,1 Gew.-% bis 10 Gew.-% der Zusammensetzung vorhanden ist, die Vitamin-B3-Verbindung in einer Konzentration von 0,1 Gew.-% bis 25 Gew.-% der Zusammensetzung vorhanden ist und die Panthenol-Verbindung in einer Konzentration von 0,01 Gew.-% bis 3 Gew.-% der Zusammensetzung vorhanden ist.

5. Zusammensetzung nach einem der Ansprüche 1-4, wobei die Xanthin-Verbindung Koffein ist, die Vitamin-B3-Verbindung Niacinamid ist und eine Panthenol-Verbindung Panthenol ist.

6. Zusammensetzung nach den Ansprüchen 1 bis 5, wobei es sich bei dem Verdickungsmittel um Acrylate/C10-30-Alkylacrylate-Kreuzpolymer handelt.

7. Zusammensetzung nach den Ansprüchen 1-6, wobei die Zusammensetzung ferner einen zusätzlichen vorteilhaften Wirkstoff enthält, der aus der Gruppe bestehend aus Mitteln zum Erhöhen des Haarvolumens, Haarkonditioniermitteln, kationischen Polymeren, Silikon und Fettverbindungen, Kopfhautpflegemitteln, Haarfärbemitteln, Duftstoffen oder deren Mischungen ausgewählt ist.

8. Zusammensetzung nach den Ansprüchen 1-7, wobei die Zusammensetzung ferner andere zusätzliche Bestandteile enthält, die aus der Gruppe bestehend aus Haarwuchsmitteln, Anti-Schuppen-Mitteln, Konditioniermitteln, Vitaminen, hydrolysierten Keratinen, Proteinen, Pflanzenextrakten, Nährstoffen, Konservierungsmitteln, pH-Wert-Reglern, Salzen, Färbemitteln, Duftstoffen, Maskierungsmitteln, Ultraviolett- und Infrarotfilter- und -absorptionsmitteln oder deren Mischungen ausgewählt sind.

9. Zusammensetzung nach den Ansprüchen 1-8, wobei die Zusammensetzung keine Emulsion ist.

10. Zusammensetzung nach den Ansprüchen 1-9, wobei die Zusammensetzung nicht ausgespült werden muss.

11. Verfahren zum Erhöhen des Haardurchmessers, das topische Auftragen einer Zusammensetzung auf einen Bereich, in dem die Erhöhung des Haarvolumens gewünscht ist, umfassend; wobei die Zusammensetzung eine Haarpflegezusammensetzung ist, die Folgendes umfasst:
(i) ein Mittel zur Erhöhung des Haardurchmessers, das eine Xanthin-Verbindung, eine Vitamin-B3-Verbindung und eine Panthenol-Verbindung umfasst; und
(ii) einen Träger, der Wasser und einwertige Alkohole mit 1 bis 6 Kohlenstoffen umfasst;
wobei der einwertige Alkohol in der Zusammensetzung in einer Konzentration von 30 bis 90 Gew.-% vorhanden ist;
wobei die Xanthin-Verbindung aus Koffein-Xanthin, 1-Methyl-Xanthin, Theophyllin, Theobromin und deren Mischungen ausgewählt ist;
wobei die Vitamin-B3-Verbindung aus Niacinamid und dessen Salzen, Nicotinsäure und deren Salzen, Nicotinylalkohol und dessen Salzen, Nicotinsäureestern, Nicotinylaminosäuren, Nicotinylestern von Carbonsäuren, Nicotinsäure-N-oxid, Niacinamid-N-oxid und deren Mischungen ausgewählt ist;
wobei die Panthenol-Verbindung aus D-Panthenol, DL-Panthenol, Panthenyltriacetat, Panthetin, Pantothein, Panthenylethylether und deren Mischungen ausgewählt ist;
wobei die Zusammensetzung ferner ein Verdickungsmittel umfasst, bei dem es sich um ein nicht auf Cellulose basierendes amphiphiles Polymer handelt;
wobei die Zusammensetzung ferner ein im Wesentlichen lösliches Silikon-Konditioniermittel, bei dem es sich um ein im Wesentlichen lösliches Silikon-Copolyol-Konditioniermittel handelt, umfasst; und
wobei die Zusammensetzung 0,1 oder weniger Gew.-% im Wesentlichen unlöslicher öliger Verbindungen enthält.

12. Verwendung einer Zusammensetzung zur Erhöhung des Haarvolumens; wobei die Zusammensetzung eine Haarpflegezusammensetzung ist, die Folgendes umfasst:
(i) ein Mittel zur Erhöhung des Haardurchmessers, das eine Xanthin-Verbindung, eine Vitamin-B3-Verbindung und eine Panthenol-Verbindung umfasst; und
(ii) einen Träger, der Wasser und einwertige Alkohole mit 1 bis 6 Kohlenstoffen umfasst;
wobei der einwertige Alkohol in der Zusammensetzung in einer Konzentration von 30 bis 90 Gew.-% vorhanden ist;
wobei die Xanthin-Verbindung aus Koffein-Xanthin, 1-Methyl-Xanthin, Theophyllin, Theobromin und deren Mischungen ausgewählt ist;
wobei die Vitamin-B3-Verbindung aus Niacinamid und dessen Salzen, Nicotinsäure und deren Salzen, Nicotinylalkohol und dessen Salzen, Nicotinsäureestern, Nicotinylaminosäuren, Nicotinylestern von Carbonsäuren, Nicotinsäure-N-oxid, Niacinamid-N-oxid und deren Mischungen ausgewählt ist;
wobei die Panthenol-Verbindung aus D-Panthenol, DL-Panthenol, Panthenyltriacetat, Panthetin, Pantothein, Panthenylethylether, Pantoyllactose, Ethylpanthenol und deren Mischungen ausgewählt ist.

## Revendications

1. Composition pour soins capillaires comprenant :
(i) un agent d'augmentation de diamètre de cheveux comprenant un composé de xanthine, un composé de vitamine B3 et un composé de panthénol ; et
(ii) un véhicule comprenant de l'eau et des alcools monohydriques ayant 1 à 6 carbones ;
dans laquelle l'alcool monohydrique est présent dans la composition à un taux en poids allant de 30 % à 90 %.
dans laquelle ledit composé de xanthine est choisi parmi la caféine-xanthine, la 1-méthyl-xanthine, la théophylline, la théobromine, et des mélanges de ceux-ci ;
dans laquelle ledit composé de vitamine B3 est choisi parmi le niacinamide et des sels de celui-ci, l'acide nicotinique et des sels de celui-ci, l'alcool nicotinylique et des sels de celui-ci, des esters d'acide nicotinique, des acides aminés de nicotinyle, des esters d'alcool nicotinylique d'acides carboxyliques, un N-oxyde d'acide nicotinique, un N-oxyde de niacinamide, et des mélanges de ceux-ci ;
dans laquelle ledit composé de panthénol est choisi parmi le D-panthénol, le DL-panthénol, le triacétate de panthényle, la panthétine, la pantothéine, l'éther éthylique de panthényle, le pantoyl-lactose, l'éthyl-panthénol, et des mélanges de ceux-ci ;
où la composition comprend en outre un épaississant qui est un polymère amphiphile n'étant pas à base de cellulose ;
où la composition comprend en outre un agent de conditionnement siliconé essentiellement soluble qui est un agent de conditionnement copolyol de silicone essentiellement soluble ;
où la composition inclut 0,1 % ou moins en poids de composés huileux essentiellement insolubles.

2. Composition selon la revendication 1, dans laquelle l'alcool monohydrique est choisi dans le groupe constitué d'éthanol, isopropanol, et des mélanges de ceux-ci.

3. Composition selon l'une quelconque des revendications 1 à 2, dans laquelle l'alcool monohydrique est l'éthanol.

4. Composition selon l'une quelconque des revendications 1 à 3, dans laquelle le composé de xanthine est présent dans la composition à un taux allant de 0,1 % à 10 % en poids de la composition, le composé de vitamine B3 est présent dans la composition à un taux allant de 0,1 % à 25 % en poids de la composition, et le composé de panthénol est présent dans la composition à un taux allant de 0,01 % à 3 % en poids de la composition.

5. Composition selon l'une quelconque des revendications 1 à 4, dans laquelle le composé de xanthine est la caféine, le composé de vitamine B3 est le niacinamide, et un composé de panthénol est le panthénol.

6. Composition selon les revendications 1 à 5, dans laquelle l'épaississant est un polymère réticulé acrylates/acrylate d'alkyle en C10 à 30.

7. Composition selon les revendications 1 à 6, où la composition contient en outre un agent à effet bénéfique supplémentaire choisi dans le groupe constitué d'agents d'augmentation de volume des cheveux, agents de conditionnement des cheveux, polymères cationiques, composés de silicone et gras, agents de sensation pour le cuir chevelu, agents de coloration capillaire, agents parfumés, ou des mélanges de ceux-ci.

8. Composition selon les revendications 1 à 7, où la composition comprend en outre d'autres composants supplémentaires choisis dans le groupe constitué d'agents pour la pousse des cheveux, agents anti-pelliculaires, agents de conditionnement, vitamines, kératines hydrolysées, protéines, extraits de plante, nutriments, conservateurs, agents d'ajustement du pH, sels, agents colorants, parfums, agents séquestrants, agents d'écran et d'absorption des ultraviolets et infrarouges, ou des mélanges de ceux-ci.

9. Composition selon les revendications 1 à 8, où la composition est sous une forme autre qu'une émulsion.

10. Composition selon l'une quelconque des revendications 1 à 9, où la composition est pour une utilisation sans rinçage.

11. Procédé pour augmenter le diamètre de cheveux, comprenant l'application topique d'une composition à une région où une augmentation de diamètre de cheveux est souhaitée ; où la composition est une composition pour soins capillaires comprenant :
(i) un agent d'augmentation de diamètre de cheveux comprenant un composé de xanthine, un composé de vitamine B3 et un composé de panthénol ; et
(ii) un véhicule comprenant de l'eau et des alcools monohydriques ayant 1 à 6 carbones ;
dans laquelle l'alcool monohydrique est présent dans la composition à un taux en poids allant de 30 % à 90 % ;
dans laquelle ledit composé de xanthine est choisi parmi la caféine-xanthine, la 1-méthyl-xanthine, la théophylline, la théobromine, et des mélanges de ceux-ci ;
dans laquelle ledit composé de vitamine B3 est choisi parmi le niacinamide et des sels de celui-ci, l'acide nicotinique et des sels de celui-ci, l'alcool nicotinylique et des sels de celui-ci, des esters d'acide nicotinique, des acides aminés de nicotinyle, des esters d'alcool nicotinylique d'acides carboxyliques, un N-oxyde d'acide nicotinique, un N-oxyde de niacinamide, et des mélanges de ceux-ci ;
dans laquelle ledit composé de panthénol est choisi parmi le D-panthénol, le DL-panthénol, le triacétate de panthényle, la panthétine, la pantothéine, l'éther éthylique de panthényle, le pantoyl-lactose, l'éthyl-panthénol, et des mélanges de ceux-ci ;
où la composition comprend en outre un épaississant qui est un polymère amphiphile n'étant pas à base de cellulose ;
où la composition comprend en outre un agent de conditionnement siliconé essentiellement soluble qui est un agent de conditionnement copolyol de silicone essentiellement soluble ; et
où la composition inclut 0,1 % ou moins en poids de composés huileux essentiellement insolubles.

12. Utilisation d'une composition pour augmenter le diamètre des cheveux ; où la composition est une composition pour soins capillaires comprenant :
(i) un agent d'augmentation de diamètre de cheveux comprenant un composé de xanthine, un composé de vitamine B3 et un composé de panthénol ; et
(ii) un véhicule comprenant de l'eau et des alcools monohydriques ayant 1 à 6 carbones ;
dans laquelle l'alcool monohydrique est présent dans la composition à un taux en poids allant de 30 % à 90 % ;
dans laquelle ledit composé de xanthine est choisi parmi la caféine-xanthine, la 1-méthyl-xanthine, la théophylline, la théobromine, et des mélanges de ceux-ci ;
dans laquelle ledit composé de vitamine B3 est choisi parmi le niacinamide et des sels de celui-ci, l'acide nicotinique et des sels de celui-ci, l'alcool nicotinylique et des sels de celui-ci, des esters d'acide nicotinique, des acides aminés de nicotinyle, des esters d'alcool nicotinylique d'acides carboxyliques, un N-oxyde d'acide nicotinique, un N-oxyde de niacinamide, et des mélanges de ceux-ci ;
dans laquelle ledit composé de panthénol est choisi parmi le D-panthénol, le DL-panthénol, le triacétate de panthényle, la panthétine, la pantothéine, l'éther éthylique de panthényle, le pantoyl-lactose, l'éthy-panthénol, et des mélanges de ceux-ci.
